(19) Europäisches Patentamt
European Patent Office
Office européen des brevets

(11) Publication number: 0 162 568 A2

# (12) EUROPEAN PATENT APPLICATION

(21) Application number: 85302616.9

(22) Date of filing: 15.04.85

(51) Int. Cl.[4]: C 07 H 15/26
A 61 K 31/70, C 12 P 19/58
//(C12P19/58, C12R1:465)

(30) Priority: 24.04.84 US 603394

(43) Date of publication of application: 27.11.85 Bulletin 85/48

(84) Designated Contracting States: BE CH DE FR GB IT LI NL SE

(71) Applicant: THE UPJOHN COMPANY
301 Henrietta Street
Kalamazoo, Michigan 49001(US)

(72) Inventor: Argoudelis, Alexander Demetrios
c/o The Upjohn Company 301 Henrietta Street
Kalamazoo Michigan 49001(US)

(72) Inventor: Marshall, Paul
c/o The Upjohn Company 301 Henrietta Street
Kalamazoo Michigan 49001(US)

(72) Inventor: Shilliday, Franklin Barclay
c/o The Upjohn Company 301 Henrietta Street
Kalamazoo Michigan 49001(US)

(72) Inventor: Bannister, Brian
c/o The Upjohn Company 301 Henrietta Street
Kalamazoo Michigan 49001(US)

(74) Representative: Perry, Robert Edward et al,
GILL JENNINGS & EVERY 53-64 Chancery Lane
London WC2A 1HN(GB)

## (54) Paulomycin analogues and their preparation.

(57) Paulomycin analogues of the formula

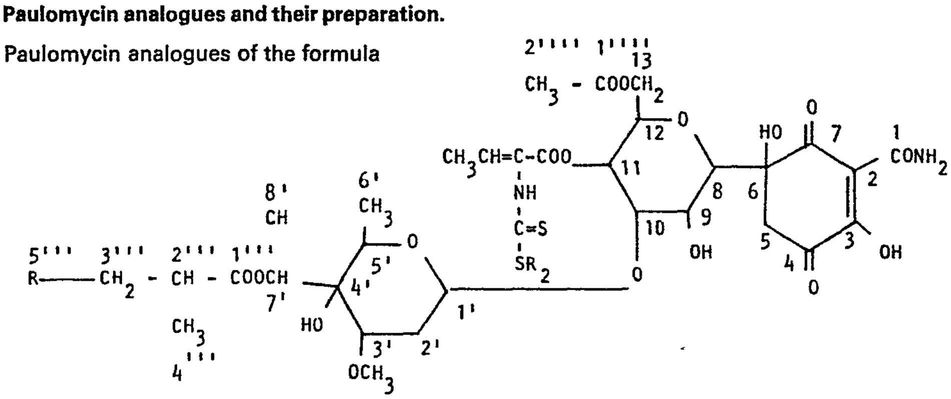

wherein R is H or $CH_3$ and $R_2$ is a substituent bearing a carboxyl/amine/hydroxyl group.

EP 0 162 568 A2

# PAULOMYCIN ANALOGUES AND THEIR PREPARATION

The present invention relates to analogues of the antibiotic paulomycin, and to their preparation.

EP-A-0046641 discloses the antibiotics which are named therein as volonomycin A and volonomycin B, but which are now generally known as paulomycin A and paulomycin B. These antibiotics are produced by fermentation of Streptomyces paulus, strain 273, NRRL 12251. Paulomycin, from which the A and B antibiotics can be isolated, is relatively insoluble in water.

EP-A-0124232 discloses antibiotics $273a_1$, $273a_{1\alpha}$ and $273a_{1\beta}$, the first of which is a mixture from which the second and third can be resolved.

Novel compounds according to the present invention are of formula I (and include the pharmacologically acceptable salts thereof)

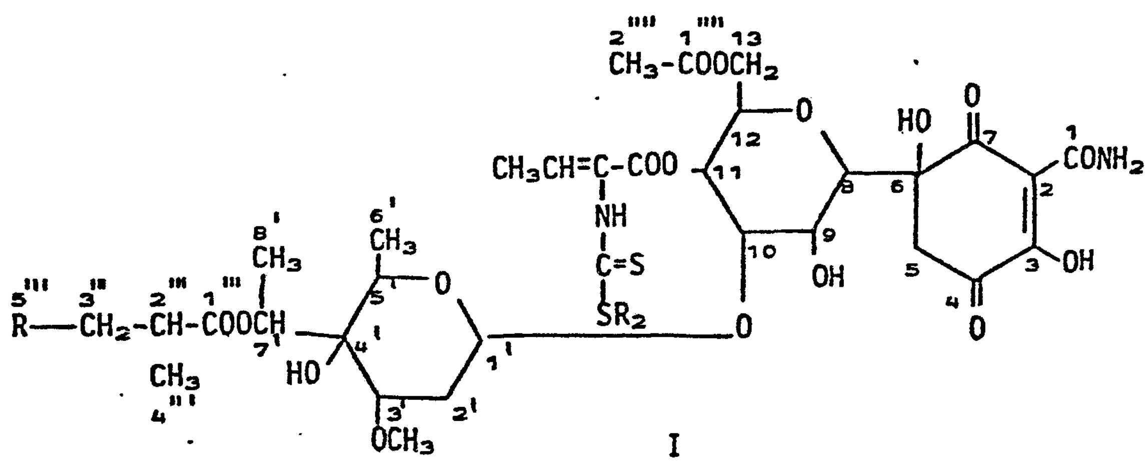

I

wherein R is hydrogen or methyl;

wherein $R_2$ is

(a) $-CH_2CH(COOX_1)-NH-COCH_3$,

(b) $-CH_2COOX_2$,

(c) $CH_3CHCOOX_2$,

(d) $COOX_2$
$CH-$
$CH_2$
$CHOOX_2$,

(e) $-CH_2CHCOOX_2$
$NH_2$,

(f) $-CH_2CH_2CHCOOX_2$
$NH_2$,

(g) $H_2NCHCH_2CH_2CONHCHCONHCH_2COOX_2$
$COOX_2$ $CH_2-$,

(h) 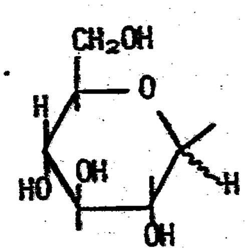

(i) $CH_2-$
$CHOH$
$CH_2OH$, or

(j) $CH_2-$
$[CHOH]_n$
$CH_2OH$;

wherein n is 3 or 4;

wherein $X_1$ is

(a) hydrogen,

(b) $(C_1-C_{12})$alkyl, or

(c) a pharmacologically acceptable cation; and

wherein $X_2$ is

(a) hydrogen, or

(b) a pharmacologically acceptable cation.

The present invention also provides:

(1) a process for preparing antibiotic $273a_2$ which comprises:

(a) extracting the clear filtrates of <u>Streptomyces paulus</u> Strain 273 (NRRL 12251) with ethyl acetate at a pH of approximately 3.0;

(b) adjusting the pH of the extract to approximately 5.5;

(c) recovering antibiotic $273a_2$ from the resultant aqueous phase; and

(d) separating antibiotics $273a_1$ and $273a_2$ by counter current distribution or HPLC;

(2) a process for preparing compounds of claim 1, wherein $R_2$ is

$-CH_2CHCOOH$,
  |
  $NHCOCH_3$,

which comprises reacting approximately equimolar amounts of N-acetyl-L-cysteine and paulomycins A and B; and

(3) a process for preparing a compound of Chart IV which comprises reacting Paulomycins A or B with a compound of the formula $R_2SH$.

The carbon atom content of various hydrocarbon-containing moieties is indicated by a prefix designating the minimum and maximum number of carbon atoms in the moiety, i.e., the prefix $(C_i-C_j)$ indicates a moiety of the integer "i" to the integer "j" carbon atoms, inclusive. Thus $(C_1-C_3)$alkyl refers to alkyl of one to 3 carbon atoms, inclusive, or methyl, ethyl, propyl, and isopropyl.

Examples of alkyl of one to 12 carbon atoms, inclusive, are methyl, ethyl, propyl, butyl, pentyl, hexyl, heptyl, octyl, nonyl, decyl, undecyl, dodecyl, and isomeric forms thereof.

Pharmacologically acceptable cations within the scope of $X_1$ include pharmacologically acceptable metal cations, ammonium, amine cations, or quarternary ammonium cations.

Especially preferred metal cations are those derived from the alkali metals, e.g., lithium, sodium, and potassium, and from the alkaline earth metals, e.g., aluminum, zinc, and iron are within the scope of this invention.

Pharmacologically acceptable amine cations are those derived from primary, secondary, or tertiary amines. Examples of suitable amines are methylamine, dimethylamine, trimethylamine, ethylamine, dibutylamine, triisopropylamine, M-methylhexylamine, decylamine, dodecylamine, allylamine, crotylamine, cyclopentylamine, dicyclohexylamine, benzyla-

mine, dibenzylamine, α-phenylethylamine, β-phenylethylamine, ethylenediamine, diethylenetriamine, and the like, aliphatic, cycloaliphatic, araliphatic amines containing up to and including about 18 carbon atoms, as well as heterocyclic amines, e.g., piperidine, morpholine, pyrrolidine, piperazine, and lower-alkyl derivatives thereof, e.g., 1-methylpiperidine, 4-ethylmorpholine, 1-isopropylpyrrolidine, 2-methylpyrrolidine, 1,4-dimethylpiperazine, 2-methylpiperidine, and the like, as well as amines containing water-solubilizing or hydrophilic groups, e.g., mono-, di-, and triethanolamine, ethyldiethanolamine, N-butylethanolamine, 2-amino-1-butanol, 2-amino-2-ethyl-1,3-propanediol, 2-amino-2-methyl-1-propanol, tris(hydroxymethyl)aminomethane, N-phenylethanolamine, N-(p-tert-amylphenyl)-diethanolamine, glactamine, N-methylglycamine, N-methylglucosamine, ephedrine, phenylephrine, epinephrine, procaine, and the like. Further useful amine salts are the basic amino salts, e.g., lysine and arginine.

Examples of suitable pharmacologically acceptable quaternary ammonium cations are tetramethylammonium, tetraethylammonium, benzyltrimethylammonium, phenyltriethylammonium, and the like.

Antibiotics $273a_2$ have been discovered in the fermentation broth of Streptomyces paulus which also produces the known antibiotics paulomycin A and paulomycin B. The structures of paulomycin A, paulomycin B and antibiotic $273a_2$ are shown in Chart I.

Antibiotics $273a_2\alpha$ and $273a_2\beta$ can also be obtained by reaction of paulomycins A and B, respectively, with N-acetyl-L-cysteine, as disclosed herein. The antibiotics $273a_2$ thus formed, which are dibasic acidic compounds, may then be reacted with appropriate organic or inorganic bases to form mono-, or di-, cation salts. Further, paulomycin A and B can react with a variety of esters of N-acetyl-L-cysteine, e.g., methyl, ethyl, butyl and octyl, to give antibacterially-active ester compounds wherein $X_1$ is $(C_1$-$C_{12})$alkyl. Still further, paulomycin A and B, when reacted with a variety of compounds, for example, mercaptoacetic acid (5), mercaptopropionic acid (6), thiomalic acid (7), cysteine (8), homocysteine (9), glutathione (10), thioglucose (11) thioglycerol (12), 1-deoxy-1-thio-pentitol (13), and 1-deoxy-1-thio-hexitol (14), shown in Chart III, to yield antibacterially-active compounds as shown in Chart IV. Salts of these compounds may also be prepared. These procedures are described more fully below. The compounds thus prepared are used in the same

manner as the paulomycins.

The conditions for the reaction of paulomycins A and B with the mercapto compounds for the production of these Chart III products are identical to those described for the production of antibiotics $273a_2\alpha$ and $273a_2\beta$. The mercapto-compound is dissolved in phosphate buffer. The pH is adjusted to approximately 9.0; paulomycins A or B are then added under stirring (ratio of mercapto-compound to paulomycins is approximately 1.5:1). The reaction is stopped at about 30 minutes. At this time the resulting addition productions are extracted from the reaction mixture with ethyl acetate or 1-butanol at the appropriate pH's (usually about 4.0). The residue obtained after removal of the solvent is a mixture of the corresponding mono and bis adducts. Purification and separation of the desired mono adduct is obtained by chromatography over silica gel using methanol-chloroform mixtures or by reverse phase chromatography in C-18 or C-8 silica gel using acetonitrile-pH 5.5 phosphate buffer mixtures. Counter double current distribution using cyclohexane-ethyl acetate-acetone-water (1:1:1:1) can be used for the purification of the mono adducts 5a to 14b. The products of the reaction can be characterized by fast atom bombardment mass spectrometry. The compounds thus produced by addition of 1 molecule of mercapto compound to paulomycins A or B have biological properties similar to those of antibiotics $273a_2$, i.e. they are active against gram-positive organisms including Staphylococcus aureus resistant to methicillin, lincosaminide and macrolide antibiotics.

DETAILED DESCRIPTION OF THE INVENTION

The present invention is seen more fully by the examples given below.

Assay and Testing Procedures

Antibiotic production and purification was measured by a microbiological disc-plate assay procedure with Micrococcus luteus as the assay organism.

Thin-layer Chromatographic Procedures

The reaction of paulomycin with N-acetyl-L-cysteine was followed by thin-layer chromatography on silica gel G using chloroform-ethanol-water (25:30:5 v/v) or on cellulose using pH 7.0 phosphate buffer as the solvent systems. The antibiotics present in reaction mixtures in preparations obtained during purification were detected by biautography on M. luteus--seeded trays.

Spectroscopic Methods

Proton magnetic resonance spectra were recorded on a Varian XL-200 spectrometer operating at 200 MHz. Solutions (ca. 0.4 ml, ca. 0.25 M) of the compounds in $d_6$-dimethylsulfoxide or $d_6$-acetone were used. Carbon magnetic resonance spectra were recorded on a Varian CFT-80 spectrometer operating at 20.0 MHz. PMR and CMR chemical shifts are reported as ppm relative to tetramethylsilane. Mass spectra were obtained on a ZAB-2F high resolution mass spectrometer using a fast atom bombardment (FAB) source.

High-Performance Liquid Chromatography (HPLC)

All HPLC chromatography was carried out on a Hewlett-Packard Model 1084B (Hewlett-Packard, Avondale, California) instrument equipped with an HP model 79875A variable wave length detector and operating in the dual pump mode. A Brownlee 10 cm x 4.6 mm stainless steel column packed with $C_{18}$ (10μ) reverse phase was used. Mobile phases were prepared using Burdick and Jackson distilled in glass solvents. All samples and aqueous phases were filtered through a 0.45 micron filter. The mobile phases used consisted of acetonitrile - pH 5.5 0.1 M phosphate buffer (50:50 or 45:55 v/v). Samples were prepared as 1 mg/ml solutions in the initial mobile phase. Injection volume was 50 μl.

The following examples are illustrative of the processes and products of the invention, but are not to be construed as limiting. All percentages are by weight and all solvent mixture proportions are by volume unless otherwise noted.

Example 1 Preparation of Antibiotics $273a_1$ and $273a_2$ - Fermentation Method

A. Fermentation

A biologically pure culture of *Streptomyces paulus* strain 273, NRRL12251, is used to inoculate a series of 500-ml. Erlenmeyer seed flasks, each containing 100 ml. of sterile seed medium consisting of the following ingredients:

| | |
|---|---|
| Glucose monohydrate | 25 g/l. |
| Pharmamedia* | 25 g/l. |
| Tap water q.s. | 1 liter |

*Pharmamedia is an industrial grade of cottonseed flour produced by Traders Oil Mill Company, Fort Worth, Texas.

The seed medium presterilization pH is 7.2. The seed inoculum is grown for 2 days at 28°C. on a Gump rotary shaker operating at 250 r.p.m. and having a 2-1/2 inch stroke.

Seed inoculum (5%), prepared as described above, is used to inoculate a series of 500 ml. fermentation flasks containing 100 ml. of sterile fermentation medium consisting of the following ingredients:

| | |
|---|---|
| Malt extract | 30 g/l. |
| Cerelose | 10 g/l. |
| Soybean meal | 12 g/l. |
| Cornsteep liquor | 5 g/l. |
| UCON | 5 g/l. |
| Tap water q.s. | 1 liter |

Note: pH was adjusted to 7.2 before sterilization.

The inoculated fermentation flasks are incubated at a temperature of 25°C. for 3 to 5 days while being shaken on a Gump rotary shaker operating at 250 r.p.m. and having a 2-1/2 inch stroke. Foaming in the fermentation flasks is controlled by the antifoam agent UCON (a synthetic defoamer produced by Union Carbide, N.Y., N.Y.).

B. Antibiotic Assay

Fermentation beers are sedimented by centrifugation at *ca.* 3000Xg. The supernatant fluids (clear beers) are assayed for antibiotic activity vs. *S. lutea*, UC>130 using bioautographic or disc-plate methods. For bioautography, thin layer chromatography (tlc) is performed on Brinkman silica gel (Sil NHr plates) or on Brinkman cellulose (Cel 400) developed respectively in chloroform, ethanol and water (25:30:5) or in 0.1 M potassium phosphate, pH 7. Clear beer anti-*S. lutea* biounit titers are obtained by a standard disc-plate assay.

A biounit (BU) is defined as the concentration of the antibiotic which gives a 20 mm zone of inhibition under the above assay conditions. Thus, if for example a fermentation beer, or other solution containing the antibiotic, needs to be diluted 1/100 to give a 20 mm zone of inhibition, the potency of such beer or solution is 100 BU per ml.

C. Isolation of Paulomycins and Antibiotics 273a ($273a_1$, $273a_2$) from Fermentation of Streptomyces Paulus

The whole beer (500 L) was filtered using filter aid. The

filtered beer (approximately 4500 L) was adjusted to pH 3.0 with 6 N aqueous sulfuric acid. The acidified solution was extracted with 100 L of Skellysolve B (a commercial mixture of essentially n-hexane); the extract was found bioinactive and was discarded. The acidic clear filtrate was then extracted twice with 1500 L of ethyl acetate. The ethyl acetate extracts were combined and washed with 1000 L of water at pH 5.3 (AQUEOUS-1). The ethyl acetate extract was then concentrated to a volume of approximately 40 L and poured into 800 L of Skellysolve B under stirring. The precipitate was isolated by filtration and dried. (Preparation A, 95.4 g). The Skellysolve B filtrate was concentrated to an oil residue (preparation B, 269 g). The aqueous solution, designated as AQUEOUS-1, was adjusted to pH 3.0 with aqueous hydrochloric acid and was extracted twice with 300 L of ethyl acetate. The ethyl acetate extracts were concentrated to a volume of 24 L and this solution mixed with 250 L of Skellysolve B. The precipitated material was isolated by filtration (Preparation C, 176.5 g). The filtrate was concentrated to dryness to give Preparation D, 39.6 g. Assay vs. M. Luteus showed the following results.

| | |
|---|---|
| Prep A | 160 bu/mg |
| Prep B | 1 bu/mg |
| Prep C | 52 bu/mg |
| Prep D | 2 bu/mg |

Preparation A contained (by tlc) paulomycins and small amounts of antibiotic $273a_2$. Preparation C contained paulomycins and antibiotics $273a_1$ and $273a_2$ as the main components. Preparations B and D were discarded.

Preparation C was used for the isolation of antibiotics 273a as described below.

D. Silica Gel Chromatography. Separation of Paulomycin and Antibiotics $273a_1$ and $273a_2$

1. Preparation of Silica Gel

One kg of silica gel (Merck-Darmstadt 7734) was triturated with 800 ml of a solution containing 38 g of KCl per L, adjusted to pH 2.0 with 1N aqueous hydrochloric acid. The KCl-HCl treated silica was activated by heating at 110° for 20 hours.

2. Preparation of the Starting Material

Preparation C 115 g, isolated as described earlier, was dissolved in chloroform-ethanol-water (25:30:5 v/v); final solution, 500 ml; approximately 230 mg/ml. Part of this solution was used as the starting material for the chromatography described below.

3. Chromatography

Activated KCl-HCl-treated silica gel, prepared as described above, was packed into a glass column using chloroform-ethanol-water (25:30:5) as the solvent system. The starting material, 43.5 ml (containing approximately 10 g of preparation C) was added on the top of the column and was adsorbed on the silica bed. The column was eluted with the chloroform-ethanol-water system at the rate of 20 ml/minute. Fractions of 20 ml were collected and tested for bioactivity against M. luteus. Results are presented below.

| Fraction No. | Zone (mm) | Fraction No. | Zone (mm) |
|---|---|---|---|
| 20 | 0 | 420 | 33 |
| 40 | 0 | 440 | 32.5 |
| 60 | 0 | 460 | 32.5 |
| 80 | 31.5 | 480 | 31 |
| 100 | 30 | 500 | 21 |
| 120 | 28 | 520 | 30 |
| 140 | 22 | 540 | 28.5 |
| 160 | 18.5 | 560 | 26.5 |
| 180 | 26 | 580 | 25 |
| 200 | 21 | 600 | 23 |
| 220 | 20 | 620 | 19 |
| 240 | 20 | 640 | 18 |
| 260 | 19 | 660 | 16 |
| 280 | 19 | 680 | 15 |
| 300 | 19 | 700 | 0 |
| 320 | 19.5 | 720 | 0 |
| 340 | 22 | | |
| 360 | 26 | | |
| 380 | 29.5 | | |
| 400 | 32 | | |

Thin layer chromatographic analysis of the isolated fractions indicated the following:

| Fractions | Compound |
|---|---|
| 80 | Paulomycin |
| 100 | $273a_2$ and small amount of paulomycin |
| 120-180 | $273a_2$ |
| 200 | $273a_2$ (traces) |
| 220-320 | no tlc spots |
| 340-600 | $273a_1$ only |

Fractions 65-140 containing antibiotic $237a_2$ and paulomycin were concentrated to dryness to give preparation E, 10.07 g.

Two additional chromotographies run under identical conditions gave preparations F, 10.0 g and G, 6.61 g.

Preparations F, G, and E were combined and this material was distributed between 500 ml of water and 500 ml of ethyl acetate at pH 3.0. The ethyl acetate phase was separated and kept as ethyl acetate -1; the aqueous phase was extracted twice with 250 ml portions of ethyl acetate (ethyl acetate -2 and ethyl acetate -3). The three ethyl acetate extracts were combined and concentrated to dryness to give preparation H. TLC showed the presence of paulomycin and antibiotic $237a_2$; preparation H was further purified as described below.

E. Silica Gel Chromatography, Separation of Antibiotic $273a_2$ from Paulomycin

1. Preparation of Silica Gel

One kg of silica gel (Merck-Darmstadt 7734) was triturated with 800 ml of a solution containing 38 g of KCl per L, adjusted to pH 2.0 with 1 M aqueous hydrochloric acid. The KCl-HCl treated silica was activated by heating at 110° for 20 hours. The column was prepared by mixing buffered silica gel with methyl ethyl ketone-acetone-water (160:50:20 v/v).

2. Preparation of the Starting Material

Preparation H, isolated as described earlier, was dissolved in 55 ml of methyl ethyl ketone, 17 ml of acetone and 6.6 ml of water. The solution was added on the top of the column. The column was eluted with the system consisting of methyl ethyl ketone-acetone-water (165:50:20 v/v) at the rate of 10 ml/minute. Fractions of 20 ml were collected and tested for bioactivity against M. luteus. Results are presented below.

| Fraction No. | Zone (mm) | Fraction No. | Zone (mm) |
|---|---|---|---|
| 10 | 0 | 260 | 28 |
| 20 | 0 | 270 | 28 |
| . | . | 280 | 28 |
| . | . | 290 | 27 |
| . | . | 300 | 25 |
| 60 | 0 | 310 | 24 |
| 70 | 24 | 320 | 22 |
| 80 | 31 | 330 | 21 |
| 90 | 27 | 340 | 19 |
| 100 | 24 | 350 | 17 |
| 110 | 21 | 360 | 16.5 |
| 120 | 19 | 370 | 16 |
| 130 | 19 | 380 | 15.5 |
| 140 | 18.5 | 390 | 15 |
| 150 | 19 | 400 | 15.5 |
| 160 | 19 | 410 | 15 |
| 170 | 19 | 420 | 15 |
| 180 | 19 | 430 | 15 |
| 190 | 19 | 440 | traces |
| 200 | 19.5 | 450 | traces |
| 210 | 20 | 460 | traces |
| 220 | 21 | 470 | traces |
| 230 | 22 | | |
| 240 | 26 | | |
| 250 | 27 | | |

Thin layer chromatography indicated that fractions 60-120 contained paulomycin, fractions 230-300 contained antibiotic $273a_2$ only.

Fractions 230-300 were combined and concentrated to dryness to give preparation I.

Preparation I was distributed between 200 ml of ethyl acetate and 200 ml of water at pH 3.0. The ethyl acetate extract was kept as EtOAc-1. The aqueous phase was extracted once with 100 ml of ethyl acetate (EtOAc-2). The two extracts (EtOAc-1, EtOAc-2) were combined and concentrated to dryness to give preparation J, 1.82 g.

Preparation J was dissolved in 20 ml of acetone. The solution was poured into 200 ml of ether. Precipitated material was isolated by filtration (K, 250 mg). The filtrate was mixed with 300 ml of cyclohexane to give a precipitate which was isolated by filtration and dried (L, 740 mg).

Preparation L contained, by tlc, antibiotic $273a_2$ only. The properties of antibiotic $273a_2$ are described in the characterization section.

Example 2 Preparation of Antibiotic $273a_2$ - Synthetic Method

A. Preparation of Antibiotic $273a_2$

N-Acetyl-L-cysteine, 314 mg (1.92 mmoles) was dissolved in 100 ml of 0.1 M pH 7.85 phosphate buffer. This solution was adjusted to pH 8.7 with 1 N aqueous potassium hydroxide. Paulomycin (A and B mixture), 1.0 g, (1.28 mmoles) was added to this solution and dissolved under stirring. After standing at room temperature for 20 minutes the solution was adjusted to pH 3.0 and extracted twice with 100 ml portions of ethyl acetate. The ethyl acetate extracts were combined, dried over sodium sulfate and concentrated to dryness to give preparation M, 1.17 g.

In another series of experiments an additional 1.12 g of reaction product (N) was obtained.

Preparation N and M were found by HPLC to be identical and to contain $273a_1\alpha$, $273a_1\beta$, $273a_2\alpha$ and $273a_2\beta$.

Preparation N and M were combined, dissolved in 28 ml of acetone and the acetone solution was poured into a mixture of 300 ml of ether and 150 ml of Skellysolve B. The precipitated material was isolated by filtration and dried to give preparation O, 1.56 g.

Like preparations N and M, preparation O contained both antibiotics $273a_1$ and both antibiotics $273a_2$. Separation of antibiotics $273a_1$ from antibiotics $273a_2$ was obtained by counter double current distribution described below.

B. Separation of Antibiotics $273a_1$ and $273a_2$ by Counter Double Current Distribution

Solvent system: cycloexane-ethyl acetate-acetone-water (1:1:1:1)
Starting material: preparation O, 1.5 g.

The starting material was dissolved in both phases and put in tube 35 (where the lower phase enters the machine). A total of 85 transfers were run collecting upper phase only. Thin layer chromatography indicated that fractions, upper machine 45-0 and lower machine 0-10, contained antibiotic $273a_2$. Fractions, lower machine 20-50, contained antibiotic $273a_1$.

Fractions containing antibiotic $273a_2$ were concentrated to dryness to give preparation P, 500 mg. Preparation P was dissolved in 2 ml of acetone and 2 ml of chloroform. This solution was poured, under stirring, into a mixture of 20 ml of ether and 60 ml of Skellysolve B. The precipitated material was isolated by filtration and dried to give preparation Q, 440 mg. Preparation Q contained (by

HPLC) both antibiotics $273a_2\alpha$ and $2732a_2\beta$. Characterization of preparation Q follows in the Characterization Section.

Example 3 Preparation of Antibiotic $273a_2\alpha$

Paulomycin A, 1.0 g (1.3 mmoles) was dissolved in 20 ml of acetonitrile and 20 ml of 0.1 M pH 7.5 phosphate buffer (Solution A). N-Acetyl-L-cysteine, 314 mg (1.92 mmoles) was dissolved in 60 ml of pH 7.85 buffer. The pH was adjusted to 8.7 with 1 N aqueous potassium hydroxide solution (Solution B). Solution B was then added to Solution A and the mixture was allowed to stand at room temperature for 20 minutes. It was then adjusted to pH 3.0 and extracted twice with 100 ml portions of ethyl acetate. The ethyl acetate extracts were combined, dried over sodium sulfate and concentrated to dryness to give preparation R. Preparation R, which contained $273a_1\alpha$ and $273a_2\alpha$ (by HPLC), was dissolved in 15 ml of acetone. This solution was poured into 150 ml of ether and 75 ml of Skellysolve B. The precipitated material was isolated by filtration and dried to give preparation S, 1.12 g.

A. Separation of Antibiotics $273a_1\alpha$ and $273a_2\alpha$ by Counter Double Current Distribution

Solvent system: cyclohexane-ethyl acetate-acetone-water (1:1:1:1)

Starting material: Preparation S, 1.0 g.

The starting material was dissolved in both phases and put in tube 35 (where the lower phase enters the machine). A total of 85 transfers were run collecting upper phase only. Thin layer chromatography indicated that fractions, upper machine 45-0 and lower machine 1-10, contained antibiotic $273a_2\alpha$. Fractions, lower machine 21-40, contained antibiotic $273a_1\alpha$.

Fractions containing antibiotic $273a_2\alpha$ were combined. The upper phase was separated (UPPER). The lower phase was extracted twice with 1/2 upper-phase-volume of ethyl acetate. The ethyl acetate extracts were combined with the UPPER phase and the mixture was concentrated to dryness to give preparation T. Preparation T was dissolved in 1 ml of chloroform and 1 ml of acetone. This solution was poured into a mixture of 10 ml of ether and 30 ml of Skellysolve B. The precipitated material was isolated by filtration and dried to give preparation U, 390 mg. Characterization of preparation U follows in the Characterization Section.

Example 4 Preparation of Antibiotic $273a_2\beta$

Paulomycin B, 1.0 g (1.3 mmoles) was dissolved in 20 ml of acetonitrile and 20 ml of 0.1 M pH 7.5 phosphate buffer (Solution A). N-Acetyl-L-cysteine, 314 mg (1.92 mmoles) was dissolved in 60 ml of pH 7.85 buffer. The pH was adjusted to 8.7 with 1 N aqueous potassium hydroxide solution (Solution B). Solution B was then added to Solution A and the mixture was allowed to stand at room temperature for 20 minutes. It was then adjusted to pH 3.0 and extracted twice with 100 ml portions of ethyl acetate. The ethyl acetate extracts were combined, dried over sodium sulfate and concentrated to dryness to give preparation V, 1.24 g. Preparation V, which contained $273a_1\beta$ and $273a_2\beta$ (by HPLC), was dissolved in 15 ml of acetone. This solution was poured into 150 ml of ether and 75 ml of Skellysolve B. The precipitated material was isolated by filtration and dried to give preparation W, 1.05 g.

A. Separation of Antibiotics $273a_1\beta$ and $273a_2\beta$ by Counter Double Current Distribution

Solvent system: cyclohexane-ethyl acetate-acetone-water (1:1:1:1)

Starting material: Preparation W, ~1.0 g.

The starting material was dissolved in both phases and put in tube 34 and 35 (where the lower phase enters the machine). A total of 85 transfers were run collecting upper phase only. Thin layer chromatography indicated that fractions, upper machine 50-0 contained antibiotic $273a_2\beta$. Fractions, lower machine 21-45, contained antibiotic $273a_1\beta$.

Fractions containing antibiotic $273a_2\beta$ were combined. The upper phase was separated. The lower phase was extracted twice with 1/2 upper-phase-volume of ethyl acetate. The ethyl acetate extracts were combined with the upper phase and the mixture was concentrated to dryness to give preparation X. Preparation X was dissolved in 2 ml of chloroform and 2 ml of acetone. This solution was poured into a mixture of 10 ml of ether and 50 ml of Skellysolve B. The precipitated material ($273a_2\beta$) was isolated by filtration and dried to give preparation Y, 350 mg. Characterization of preparation Y follows in the Characterization Section.

Example 5 Reaction of Paulomycins A and B with esters of N-acetyl-L-cysteine

1. Preparation of N-acetyl-L-cysteine Methyl, Ethyl, Butyl and Octyl esters

N-acetyl-L-cysteine (1 equivalent) is dissolved in excess of methyl, ethyl, butyl or octyl alcohol. Thionyl chloride (1.1 to 1.5 equivalents) is then added to the N-acetyl-L-cysteine-alcohol mixture and this mixture is allowed to stand at room temperature for 1-3 hours. The reaction mixture is then concentrated to dryness. Crystallization is obtained from ether-heptane mixture.

2. Reaction of Paulomycins (A or B) with N-acetyl-L-cysteine Esters

Paulomycin (1 equivalent) is dissolved in a mixture of tetrahydrofuran-phosphate buffer, pH 8.5. One and one-half equivalents of the corresponding N-acetyl-L-cysteine ester (methyl, ethyl, butyl, octyl) is then added under stirring. After 30 minutes at room temperature the reaction mixture is extracted with ethyl acetate. The extract is concentrated to dryness to give a residue containing both the "mono" and the "bis" adducts. Purification and isolation of the "mono" adduct is obtained by counter double current distribution as described below.

3. Purification. Counter Double Current Distribution

The solvent consists of cyclohexane-ethyl acetate-acetone-water (1:1:1:1 v/v). The residue obtained as described above is dissolved in both phases of the solvent system and added in one tube (center tube) of an all-glass counter double current distribution apparatus (100 tubes). After one hundred transfers, fractions are analyzed by thin layer chromatography. Fractions containing the "mono" adduct are concentrated to dryness. The residue is purified by precipitation from acetone-Skellysolve mixtures. Characterization is obtained by fast atom bombardment. The molecular formulas of the "mono" adducts obtained by reaction of paulomycins A and B with N-acetyl-L-cysteine esters are tabulated in Table 2.

Example 6 Reaction of paulomycin with mercaptoacetic acid, mercaptopropionic acid and thiomalic acid

1. Reaction of Paulomycins with Mercaptoacetic Acid

Mercaptoacetic acid (1.5 eq) was dissolved in pH 8.5 phosphate buffer. The pH was then adjusted to 8.7 with 1 N KOH and 1 equivalent of paulomycin (A or B) was added under stirring. After 2 hours at room temperature under stirring, the solution was adjusted to pH 3.0

and the paulomycins-mercaptoacetic acid addition compounds were isolated by extraction with methylene chloride or ethyl acetate. The extract was dried over sodium sulfate and concentrated to dryness. The residue obtained was then purified by precipitations from methylene chloride-heptane combinations and the precipitate was purified by counter double current diastribution as described above.

2. Reaction of Paulomycin with Mercaptopropionic Acid and Thiomalic Acid

The procedure described above was followed and specifically the ratio of the acids to paulomycin was about 2:1 equivalents. The precipitates obtained by the methylene chloride precipitation are analyzed by TLC and HPLC. Purification is obtained by counter double current distribution as described earlier.

The molecular formulas of the "mono" adducts obtained by reaction of paulomycins A and B with these compounds are presented in Table 3.

Example 7 Reaction of Paulomycins A and B with Cysteine, Homocysteine and Glutathione

The compounds (1.5 eq) are dissolved in pH 7.5 0.1 M phosphate buffer. The pH of the solution is adjusted to 8.7 with 1 N potassium hydroxide. Paulomycin (A or B) (1 eq) is added under stirring. The reaction is followed by thin layer chromatography.

The products of the reactions of paulomycins A and B and cysteine, homocysteine and glutathione can be extracted by butanol at pH 6.0. The butanolic extract is concentrated to dryness and the residue is then purified by chromatography over silica gel using chloroform-methanol mixtures as the mobile phase. alternatively, the residue obtained from the butanolic extract can be purified by reverse phase chromatography using C-18 or C-8 silica and acetonitrile-pH 5.5 0.1 M phosphate buffer mixtures, or by counter double current distribution as described earlier. The molecular formulas of the "mono" adducts obtained by reaction of paulomycins A and B with these compounds are presented in Table 4.

Example 8 Reaction of Paulomycin A and B with Thioglucose or Thioglycerol

Thioglucose or thioglycerol (1.5 eq) are dissolved in pH 7.85 phosphate buffer. The pH is then adjusted to 8.7 with 1 N KOH solution and 1 equivalent of paulomycin (A or B as mixture) is added under stirring. After 1 hour at room temperature the solution is

adjusted to pH 5.5 and passed over Amberlite XAD-4. The paulomycin-thioglucose or thioglycerol reaction products are absorbed on the resin and eluted with acetone. The acetone solution is concentrated to dryness. The residue was dissolved in acetone and this solution was mixed with ether-hexane mixture. The precipitated paulomycin-thioglucose or paulomycin-thioglycerol product was isolated by filtration and dried. Purification of the "mono" adducts is obtained by counter double current distribution as described earlier. The molecular formulas of the "mono" adducts obtained by reaction of paulomycin A or B with these compounds are presented in Table 5.

Example 9 Reaction of Paulomycin A and B with a 1-Deoxy-1-Thiopentitol or with a 1-Deoxy-1-Thiohexitol

The 1-deoxy-1-thio-pentitol or thio-hexitol (1.5 eq) is dissolved in pH 7.85 phosphate buffer. The pH is then adjusted to 8.7 with 1 N KOH solution and 1 equivalent of paulomycin (A or B as mixture) is added under stirring. After 1 hour at room temperature the solution is adjusted to pH 5.5 and passed over Amberlite XAD-4. The paulomycin-thio-pentitol or -hexitol reaction products are absorbed on the resin and eluted with acetone. The acetone solution is concentrated to dryness. The residue is dissolved in acetone or methylene chloride and this solution is mixed with ether-hexane mixture. The precipitated paulomycin-thio-pentitol or paulomycin-thiohexitol product is isolated by filtration and dried. Characterization of these materials is obtained by fast atom bombardment mass spectroscopy.

CHARACTERIZATION OF ANTIBIOTICS $273a_2$

Antibiotic $273a_{2\alpha}$ (Preparation U)

1. Appearance: Colorless, acidic, amorphous material

2. Solubility: Soluble in lower alcohols, ketones, ethyl acetate; less soluble in chloroform, methylene chloride; insoluble in ether and saturated hydrocarbon solvents. The free acid form is insoluble in water, but soluble in phosphate buffers at physiological pH (7.0-7.5). Salts are soluble in water.

3. Molecular formula: $C_{39}H_{55}N_3O_{20}S_2$

4. Molecular weight: Calculated: 949

Found: 949 (by FAB-MS)

5. <u>Anal:</u> <u>Calcd/Found</u>

| | |
|---|---|
| C | 49.31/49/11 |
| H | 5.79/5.91 |
| N | 4.42/4/30 |
| S | 6.74/6/63 |

6. $[\alpha]^{25}_D$ -33° (C, 0.895, MeOH)

7. <u>Melting point:</u> 119-150° with decomposition

8. <u>Infrared spectrum:</u> Tabulation of the IR bands is as follows:

| Band Freq. | Inten. | Type | Band Freq. | Inten. | Type | Band Freq. | Inten. | Type |
|---|---|---|---|---|---|---|---|---|
| 3470.8 | 52 | SH | 1458.1 | 6 | AVG M | 867.9 | 58 | AVG |
| 3413.9 | 41 | SH | 1377.1 | 8 | AVG M | 849.6 | 58 | BRD |
| 3355.1 | 34 | BRD | 1337.6 | 21 | AVG | 835.1 | 59 | AVG |
| 3268.3 | 32 | BRD | 1299.0 | 14 | AVG | 825.5 | 59 | AVG |
| 3233.6 | 33 | BRD | 1261.4 | 10 | AVG | 815.8 | 55 | SHP |
| 2953.0 | 1 | BRD M | 1190.0 | 25 | AVG | 778.2 | 52 | SH |
| 2916.3 | 0 | BRD M | 1153.4 | 19 | SH | 722.3 | 52 | AVG M |
| 2870.0 | 5 | AVG M | 1136.0 | 15 | AVG | 690.5 | 47 | SH |
| 2854.6 | 3 | AVG M | 1119.6 | 15 | AVG | 663.5 | 51 | SH |
| 2728.3 | 69 | BRD M | 1097.4 | 18 | AVG | 650.9 | 47 | SH |
| 1734.0 | 2 | AVG | 1055.0 | 19 | AVG | 636.5 | 47 | SH |
| 1659.7 | 20 | AVG | 1027.0 | 14 | AVG | 601.7 | 39 | AVG |
| 1625.9 | 22 | AVG | 993.3 | 21 | AVG | | | |
| 1575.8 | 17 | AVG | 910.3 | 46 | AVG | | | |
| 1529.5 | 28 | BRD | 893.0 | 54 | AVG | | | |

Band Freq.: Band Frequencies in wavenumbers ($cm^{-1}$)

Inten.: Intensity in percent transmittance (%T)

Data type in local peak region: BRD - Broad AVG - Average SHP - Sharp SH - Shoulder. This peak list is unedited

M: Possible interference from mineral oil

25 Strongest Peaks

| % T | Freq. |
|---|---|
| 0 | 2916.2 |
| 1 | 2953.0 |
| 2 | 1734.0 |
| 3 | 2854.5 |
| 5 | 2870.0 |
| 6 | 1458.0 |
| 8 | 1377.0 |
| 10 | 1261.3 |
| 14 | 1299.0 |
| 14 | 1027.0 |
| 15 | 1136.0 |
| 15 | 1119.5 |
| 17 | 1575.7 |
| 18 | 1097.3 |
| 19 | 1153.3 |
| 19 | 1055.0 |
| 20 | 1659.6 |
| 21 | 1337.5 |
| 21 | 993.2 |
| 22 | 1625.8 |
| 25 | 1190.0 |
| 28 | 1529.5 |
| 32 | 3268.2 |
| 33 | 3233.5 |
| 34 | 3355.0 |

Prep: Mineral Oil Mull

Tape: 118

File: 187

Max %T: 97 3759.2

%T at 3800 ($cm^{-1}$):96

Density (cm-1/pt): 0.964

9. UV spectrum (in 95% aqueous ethanol):

| λmax | a |
|---|---|
| 246 | 19.61 |
| 270sh | 11.85 |
| 320 | 9.35 |

Antibiotic $273a_{2\beta}$ (Preparation Y)

1. Appearance: Colorless, acidic, amorphous material.

2. Solubility: Soluble in lower alcohols, ketones, ethyl acetate; less soluble in chloroform, methylene chloride; insoluble in ether and saturated hydrocarbon solvents. The free acid form is insoluble in water, but soluble in phosphage buffers at physiological pH (7.0-7.5).

Salts are soluble in water.

3. Molecular formula: $C_{38}H_{53}N_3O_{20}S_2$

4. Molecular weight: Calculated: 935
Found: 935 (by FAB-MS)

5. Anal.:

| | Calcd/Found |
|---|---|
| C | 48.77/48.98 |
| H | 5.67/5.97 |
| N | 4.49/4.25 |
| S | 6.84/6.77 |

6. $[\alpha]^{25}_{D}$ -34 (C, 0.375, methanol)

7. Melting point: Decomposition begins at approximately 122.5°C.

8. Infrared spectrum: Tabulation of the IR bands is as follows:

| Band Freq. | Inten. | Type | Band Freq. | Inten. | Type | Band Freq. | Inten. | Type |
|---|---|---|---|---|---|---|---|---|
| 3468.0 | 53 | SH | 1574.8 | 21 | AVG | 993.3 | 24 | AVG |
| 3412.0 | 43 | SH | 1457.2 | 9 | AVG M | 930.6 | 59 | AVG |
| 3359.0 | 37 | BRD | 1377.1 | 12 | AVG M | 910.3 | 50 | AVG |
| 3271.2 | 37 | BRD | 1340.5 | 25 | AVG | 894.0 | 57 | AVG |
| 3236.5 | 37 | BRD | 1299.0 | 18 | AVG | 865.0 | 61 | SH |
| 2953.0 | 2 | AVG M | 1259.5 | 14 | AVG | 854.4 | 59 | AVG |
| 2920.2 | 0 | BRD M | 1230.5 | 21 | SH | 839.0 | 60 | AVG |
| 2870.0 | 9 | AVG M | 1201.6 | 29 | AVG | 816.8 | 58 | SHP |
| 2854.6 | 5 | AVG M | 1158.2 | 21 | AVG | 772.4 | 56 | AVG |
| 2729.2 | 72 | BRD M | 1134.1 | 18 | AVG | 723.3 | 55 | AVG M |
| 2031.0 | 90 | BRD | 1119.6 | 18 | AVG | 689.5 | 53 | AVG |
| 1734.0 | 3 | AVG | 1098.4 | 21 | AVG | 664.4 | 50 | SH |
| 1701.2 | 16 | SH | 1066.6 | 26 | SH | 650.0 | 49 | SH |
| 1660.7 | 23 | AVG | 1056.0 | 23 | AVG | 635.5 | 49 | SH |
| 1625.9 | 25 | AVG | 1027.0 | 17 | AVG | 602.7 | 42 | AVG |

Band Freq.: Band Frequencies in wavenumbers ($cm^{-1}$)

Inten.: Intensity in percent transmittance (%T)

Data type in local peak region: BRD - Broad AVG - Average SHP - Sharp SH - Shoulder. This peak list is unedited

M: Possible interference from mineral oil

25 Strongest Peaks

| % T | Freq. |
|---|---|
| 0 | 2920.1 |
| 2 | 2953.0 |
| 3 | 1734.0 |
| 5 | 2854.5 |
| 9 | 2870.0 |
| 9 | 1457.1 |
| 12 | 1377.0 |
| 14 | 1259.5 |
| 16 | 1701.1 |
| 17 | 1027.0 |
| 18 | 1299.0 |
| 18 | 1134.0 |
| 18 | 1119.5 |
| 21 | 1574.7 |
| 21 | 1230.5 |
| 21 | 1158.1 |
| 21 | 1098.3 |
| 23 | 1660.6 |
| 23 | 1056.0 |
| 24 | 993.2 |
| 25 | 1625.8 |
| 25 | 1340.5 |
| 26 | 1066.5 |
| 29 | 1201.5 |
| 37 | 3359.0 |

Prep: Mineral Oil Mull

Tape: 118

File: 280

Max %T: 95 3799.7

%T at 3800 ($cm^{-1}$): 95

Density (cm-1/pt): 0.964

9. UV spectrum: (In 95% aqueous ethanol)

| λmax | a |
|---|---|
| 246 | 18.88 |
| 270sh | 10.37 |
| 322 | 9.53 |

Antibiotic 273a$_2$ (Preparation q)

1. Appearance: Colorless, acidic, amorphous material

2. Solubility: Soluble in lower alcohols, ketones, ethyl acetate, less soluble in chloroform, methylene chloride, insoluble in ether and saturated hydrocarbon solvents. The free acid form is insoluble in water, but soluble in phosphate buffers at physiological pH (7.0-7.5).

Salts are soluble in water.

3. Molecular formula: Mixture of 273a$_2$α ($C_{39}H_{55}N_3O_{20}S_2$) and 273a$_2$β ($C_{38}H_{53}N_3O_{20}S_2$).

4. Molecular weight: 935 and 949

Found: 935, 949 (by FAM-MS)

5. Anal.: Calcd/Found (for a mixture of 50% 273a$_2$α and 50% 273a$_2$b)

| | |
|---|---|
| C | 49.04/49.32 |
| H | 5.73/5.97 |
| N | 4.45/4.28 |
| S | 6.79/6.64 |

6. $[\alpha]^{25}_D$-33° (C, 0.335 methanol)

7. Melting point: Decomposition begins at approximately 122.5°C

8. Infrared spectrum: Tabulation of the IR bands is as follows:

| Band Freq. | Inten. | Type | Band Freq. | Inten. | Type | Band Freq. | Inten. | Type |
|---|---|---|---|---|---|---|---|---|
| 3354.2 | 41 | BRD | 1339.5 | 27 | AVG | 894.0 | 60 | AVG |
| 3271.2 | 39 | BRD | 1299.0 | 20 | AVG | 868.9 | 64 | AVG |
| 3233.6 | 40 | BRD | 1261.4 | 16 | AVG | 854.4 | 63 | AVG |
| 2937.5 | 0 | BRD | 1200.6 | 32 | BRD | 840.9 | 65 | SH |
| 2868.1 | 4 | SH M | 1156.3 | 25 | AVG | 815.8 | 61 | SHP |
| 2854.6 | 2 | AVG M | 1136.0 | 20 | AVG | 772.4 | 58 | AVG |
| 2726.3 | 72 | BRD M | 1119.6 | 21 | AVG | 722.3 | 56 | AVG M |
| 1734.0 | 5 | AVG | 1097.4 | 24 | AVG | 690.5 | 58 | AVG |
| 1660.7 | 26 | AVG | 1055.0 | 26 | AVG | 664.4 | 54 | SH |
| 1626.9 | 28 | AVG | 1027.0 | 20 | AVG | 650.9 | 54 | SH |
| 1575.8 | 24 | AVG | 993.3 | 26 | AVG | 635.5 | 54 | SH |
| 1457.2 | 7 | AVG M | 932.5 | 62 | AVG | 601.7 | 48 | AVG |
| 1377.1 | 11 | AVG M | 910.3 | 52 | AVG | | | |

Band Freq.: Band Frequencies in wavenumbers ($cm^{-1}$)

Inten.: Intensity in percent transmittance (%T)

Data type in local peak region: BRD - Broad AVG - Average SHP - Sharp SH - Shoulder. This peak list is unedited

M: Possible interference from mineral oil

25 Strongest Peaks

| % T | Freq. |
|---|---|
| 0 | 2937.5 |
| 2 | 2854.5 |
| 4 | 2868.0 |
| 5 | 1734.0 |
| 7 | 1457.1 |
| 11 | 1377.0 |
| 16 | 1261.3 |
| 20 | 1299.0 |
| 20 | 1136.0 |
| 20 | 1027.0 |
| 21 | 1119.5 |
| 24 | 1575.7 |
| 24 | 1097.3 |
| 25 | 1156.2 |
| 26 | 1660.6 |
| 26 | 1055.0 |
| 26 | 993.2 |
| 27 | 1339.5 |
| 28 | 1626.8 |
| 32 | 1200.5 |
| 39 | 3271.1 |
| 40 | 3233.5 |
| 41 | 3354.1 |
| 48 | 601.6 |
| 52 | 910.2 |

Prep: Mineral Oil Mull

Tape: 118

File: 279

Max %T: 98 3726.4

%T at 3800 ($cm^{-1}$): 97

Density (cm-1/pt): 0.964

9. UV spectrum: (in 95% aqueous ethanol)

| λmax | a |
|---|---|
| 246 | 19.02 |
| 270sh | 10.63 |
| 321 | 9.57 |

Identity of $273a_2$ produced by S. paulus to synthetic $273a_2$

Antibiotic $273a_2$ isolated from fermentations of S. paulus was compared to "synthetic" $273a_2$ by IR, UV and TLC. Both preparations had similar UV and IR spectra and identical TLC behavior. Futhermore, the mass spectra (FAB) of both "synthetic" and "natural" $273a_2$-free acid were identical indicating the presence of two compounds ($273a_{2}\alpha$ and $273a_{2}\beta$)

with molecular weights of 949 and 935.

The structure of antibiotics $273a_{2\alpha}$ and $273a_{2\beta}$ are shown in Chart I.

## BIOLOGICAL PROPERTIES

Antibiotics $273a_2$ and its two components $273a_{2\alpha}$ and $273a_{2\beta}$ are active against Gram-positive organisms including organisms resistant to penicillins, macrolides and lincosaminides.

An example of the antibacterial spectrum of antibiotics $273a_2$ is provided in Table 1.

TABLE 1

ANTIMICROBIAL IN VITRO TESTING

MINIMUM INHIBITORY CONCENTRATION - MCG PER ML -

| Organism Name | UC | PH 6 |
|---|---|---|
| Staphylococcus aureus | 76 | 0.125 |
| Staphlyococcus aureus | 6675 | 0.25 |
| Staphlyococcus aureus | 6685 | 0.25 |
| Staphlyococcus aureus | 6694 | 0.25 |
| Staphlyococcus aureus | 3665 | 0.5 |
| Staphlyococcus epidermidis | 30031 | 0.25 |
| Staphlyococcus epidermidis | 30033 | 0.125 |
| Streptococcus faecalis | 9217 | 1 |
| Streptococcus faecalis | 30070 | 4 |
| Escherichia coli | 6674 | >128 |
| Klebsiella pneumoniae | 30090 | >128 |
| Citrobacter diversus | 30174 | >128 |
| Citrobacter freundii | 30151 | >128 |
| Serratia marcescens | 30161 | >128 |
| Enterobacter cloacae | 30080 | >128 |
| Enterobacter aerogenes | 30229 | >128 |
| Proteus vulgaris | 30264 | 128 |
| Proteus mirabilis | 30107 | >128 |
| Salmonella typhimurium | 30243 | >128 |
| Pseudomonas aeruginosa | 6674 | >128 |

Note: "UC" is a registered trademark of The Upjohn Company.

The conditions of the above test are as follows:

The antibacterial assay is a standard microplate agar assay using PYG agar, pH 6. PYG agar consists of the following ingredients:

| | |
|---|---|
| Peptone | 10 g./l. |
| Yeast extract | 5 g./l. |
| Glucose | 1 g./l. |
| Agar | 15 g./l. |
| Distilled water, q.s. | 1 l. |

---

The MIC is determined by standard methods. The inocula are overnight cultures of the test organisms, diluted so that the final population contains approximately $10^5$ cells/ml. The agar plates are incubated at 28°C to 37°C for 24 hrs. The lowest antibiotic concentration which allows no growth=MIC or minimum inhibitory concentration.

Thus, the antibacterially-active compounds of the present invention can be used for the same antibacterial purposes as paulomycin A and B. For example, the compounds of the invention can be used alone or in combination with other antibiotic agents to prevent the growth of, or reduce the number of the above described susceptible bacteria in many environments. For example, they can be used as disinfectants on various dental and medical equipment contaminated with Staphylococcus aureus. Further, they are useful in wash solutions for sanitation purposes, as in the washing of hands and in the cleaning of equipment, floors, or furnishings or contaminated rooms or laboratories; they are also useful as an industrial preservative, for example, as a bacteriostatic rinse for laundered clothes and for impregnating papers and fabrics; and they are useful for suppressing the growth of sensitive organisms in plate assays and other microbiological media. They also can be used as feed supplements to promote the growth of animals, for example, mammals, birds, fish, and reptiles.

The compounds of the subject invention are useful as antibacterial agents in suitable compositions. These compositions are preferably presented for administration to humans and animals in unit dosage forms, such as tablets, capsules, pills, powders, granules, sterile parenteral solutions or suspensions, and oral solutions or suspensions, and oil-water emulsions containing suitable quantities of the active compound in the form of the free base, or its pharmacologically acceptable salts.

For oral administration, either solid or fluid unit dosage forms can be prepared. For preparing solid compositions such as tablets, the principal active ingredient is mixed with conventional ingredients such as talc, magnesium stearate, dicalcium phosphate, magnesium aluminum silicate, calcium sulfate, starch, lactose, acacia, methylcellulose, and functionally similar materials as pharmaceutical diluents or carriers. The tablets can be laminated or otherwise compounded to provice a dosage form affording the advantage of prolonged or delayed action or predetermined successive action of the enclosed medication. for example, the tablet can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids or mixture of polymeric acids with such materials as shellac, cetyl alcohol, cellulose acetate phthalate, styrene maleic acid copolymer and the like. Alternatively, the two component system can be utilized for preparing tablets containing two or more incompatible active ingredients. Wafers are prepared in the same manner as tablets, differing only in shape and the inclusion of sucrose or other sweetener and flavor. In their simplest embodiment, capsules, like tablets, are prepared by mixing the compound of the formulation with an inert pharmaceutical diluent and filling and mixture into a hard gelatin capsule of appropriate size. In another embodiment, capsules are prepared by filling hard gelatin capsules with polymeric acid coated beads containing the active compound. Soft gelatin capsules are prepared by machine encapsulation of a slurry of the active compound with an acceptable vegetable oil, light liquid petrolatum or other inert oil.

Fluid unit dosage forms for oral administration such as syrups, elixirs, and suspensions can be prepared. The water-soluble forms of the active compound can be dissolved in an aqueous vehicle together with sugar, aromatic flavoring agents and preservatives to form a syrup. An elixir is prepared by using a hydro-alcoholic (ethanol) vehicle with suitable sweeteners such as sucrose together with an aromatic flavoring agent. Suspensions can be prepared of the

insoluble forms with a syrup vehicle with the aid of a suspending agent such as acacia, tragacanth, methylcellulose and the like.

Topical ointments can be prepared by dispersing the active compound in a suitable ointment base such as petrolatum, lanolin, polyethylene glycols, mixtures thereof, and the like. Advantageously, the compound is finely divided by means of a colloid mill utilizing light liquid petrolatum as a levigating agent prior to dispersing in the ointment base. Topical creams and lotions are prepared by dispersing the compound in the oil phase prior to the emulsification of the oil phase in water.

For parenteral administration, fluid unit dosge forms are prepared utilizing the active compound and a sterile vehicle, water being preferred. The active compound, depending on the form and concentration used, can be either suspended or dissolved in the vehicle. In preparing solutions, a water-soluble form of the active compound can be dissolved in water for injection and filter sterilized before filling into a suitable vial or ampul and sealing. Advantageously adjuvants such as a local anesthetic, preservative and buffering agents can be dissolved in the vehicle. To enhance the stability, the composition can be frozen after filling into the vial and the water removed under vacuum. The dry lyophilized powder is then sealed in the vial and an accompanying vial of water for injection is supplied to reconstitute the powder prior to use. Parenteral suspensions are prepared in substantially the same manner except that the active compound is suspended in the vehicle instead of being dissolved and sterilization cannot be accomplished by filtration. The active compound can be sterilized by exposure to ethylene oxide before suspending the sterild vehicle. Advantageously, a surfactant or wetting agent is included in the composition to facilitate uniform distribution of the active compound.

The term unit dosage form as used in the specification and claims refers to physically discrete units suitable as unitary dosages for human subjects and animals, each unit containing a predetermined quantity of active material calculated to produce the desired therapeutic effect in association with the required pharmaceutical diluent, carrier or vehicle. The specifications for the novel unit dosage forms of this invention are dictated by and directly dependent on (a) the unique characteristics of the active material and the

particular therapeutic effect to be achieved, and (b) the limitations inherent in the art of compounding such an active material for therapeutic use in humans and animals, as disclosed in detail in this specification, these being features of the present invention. Examples of suitable unit dosage forms in accord with this invention are tablets, capsules, pills, troches, suppositories, powder packets, granules, wafers, cachets, teaspoonfuls, tablespoonfuls, dropperfuls, ampuls, vials, segregated multiples of any of the foregoing, and other forms as herein described.

In addition to the administration of the active compound as the principal active ingredient of compositions for the treatment of the conditions described herein, the said compound can be included with other types of compounds to obtain advantageous combinations of properties. Such combinations include the active compound with antibiotics such as spectinomycin, chloramphenicol, novobiocin, dihydronovobiocin, tetracyclines (e.g., tetracycline, oxytetracycline and chlortetracycline), penicillins, erythromycin, kanamycin, streptomycin, neomycin, polymyxin, bacitracin, nystatin, filipin, fumagillin and endomycin to broaden the bacterial spectrum of the composition and for synergistic action against particular bacteria; steroids having anti-inflammatory activity such as hydrocortisone, prednisolone, 6α-methylprednisolone, 6α-fluoroprednisolone and the like; analgesics such as aspirin, sodium salicylate (acetylsalicylic acid)-anhydride, N-acetyl-p-aminophenyl and salicylamide; antihistamines, such as chlorpheniramine maleate, diphenylhydramine, promethazine, pyrathiazine, and the like; sulfas, such as sulfadiazine, sulfamethazine, sulfamerazine sulfacetamide, sulfadimethyloxazole, sulfamethizole, and the like; antifungals, such as undecylenic acid, sodium propionate, salicylanilide, sodium caprylate, and hexetidine; and the vitamins.

The dosage of the active compound for treatment depends on route of administration; the age, weight, and condition of the patient; and the particular disease to be treated. A dosage schedule of from about 15 to 500 mg., 1 to 4 times daily (every six hours), embraces the effective range for the treatment of most conditions for which the compositions are effective. For children, the dosage is calculated on the basis of 15 to 30 mg/kg/day to be administered every six hours. The selection of suitable patients and dosages for the treatment of

humans and animals with the compounds of this invention is readily undertaken by an ordinarily skilled physician or veterinarian.

The active compound is compounded with a suitable pharmaceutical carrier in unit dosage form for convenient and effective administration. In the preferred embodiments of this invention, the dosage units contain the compound in: 15, 30, 50, 125, 250, and 500 mg. amounts for systemic treatment; in 0.25, 0.5, 1, 2 and 5% amounts for topical or localized treatment; and 5 to 65% w/v for parenteral treatment. The dosage of compositions containing the active compound and one or more other active ingredients is to be determined with reference to the usual dosage of each such ingredient.

TABLE 2

PRODUCTS OF REACTION OF PAULOMYCIN A AND B WITH N-ACETYL-L-CYSTEINE ESTERS

| Paulomycin | Ester | Compound | Molecular Formula | Molecular Weight* |
|---|---|---|---|---|
| A | Methyl | 1a | $C_{40}H_{57}N_3O_{20}S_2$ | 963 |
| A | Ethyl | 2a | $C_{41}H_{59}N_3O_{20}S_2$ | 922 |
| A | Butyl | 3a | $C_{43}H_{63}N_3O_{20}S_2$ | 1005 |
| A | Octyl | 4a | $C_{47}H_{71}N_3O_{20}S_2$ | 1061 |
| B | Methyl | 1b | $C_{39}H_{55}N_3O_{20}S_2$ | 949 |
| B | Ethyl | 2b | $C_{40}H_{57}N_3O_{20}S_2$ | 963 |
| B | Butyl | 3b | $C_{42}H_{61}N_3O_{20}S_2$ | 991 |
| B | Octyl | 4b | $C_{46}H_{69}N_3O_{20}S_2$ | 1047 |

*Calculated

TABLE 3

PRODUCTS OF REACTION OF PAULOMYCINS A AND B WITH MERCAPTOACETIC ACID, MERCAPTOPROPIONIC ACID AND THIOMALIC ACID

| Paulomycin | Acid | Product | Molecular* Formula | Molecular Weight* |
|---|---|---|---|---|
| A | 13 | 5a | $C_{36}H_{50}N_2O_{19}S_2$ | 878 |
| A | 14 | 6a | $C_{37}H_{52}N_2O_{19}S_2$ | 892 |
| A | 15 | 7a | $C_{38}H_{52}N_2O_{21}S_2$ | 936 |
| B | 13 | 5b | $C_{35}H_{48}N_2O_{19}S_2$ | 864 |
| B | 14 | 6b | $C_{36}H_{50}N_2O_{19}S_2$ | 878 |
| B | 15 | 7b | $C_{37}H_{50}N_2O_{21}S_2$ | 922 |

*Calculated

TABLE 4

PRODUCTS OF REACTION OF PAULOMYCINS A AND B WITH CYSTEINE, HOMOCYSTEINE, AND GLUTATHIONE

| Paulomycin | Reactant | Product | Molecular* Formula | Molecular Weight* |
|---|---|---|---|---|
| A | Cysteine | 8a | $C_{37}H_{53}N_3O_{19}S_2$ | 907 |
| A | Homocysteine | 9a | $C_{38}H_{55}N_3O_{19}S_2$ | 921 |
| A | Glutathione | 10a | $C_{44}H_{63}N_5O_{23}S_2$ | 1093 |
| B | Cysteine | 8b | $C_{36}H_{51}N_3O_{19}S_2$ | 893 |
| B | Homocysteine | 9b | $C_{37}H_{53}N_3O_{19}S_2$ | 907 |
| B | Glutathione | 10b | $C_{43}H_{61}N_5O_{23}S_2$ | 1079 |

*Calculated

TABLE 5

PRODUCTS OF REACTION OF PAULOMYCIN A AND B WITH THIOGLUCOSE AND THIOGLYCCEROL

| Paulomycin | Reactant | Product | Molecular* Formula | Molecular Weight* |
|---|---|---|---|---|
| A | Thioglucose | 11a | $C_{40}H_{58}N_2O_{22}S_2$ | 982 |
| A | Thioglycerol | 12a | $C_{37}H_{54}N_2O_{19}S_2$ | 894 |
| B | Thioglucose | 11b | $C_{39}H_{56}N_2O_{22}S_2$ | 968 |
| B | Thioglycerol | 12b | $C_{36}H_{52}N_2O_{19}S_2$ | 880 |

*Calculated

CHART I

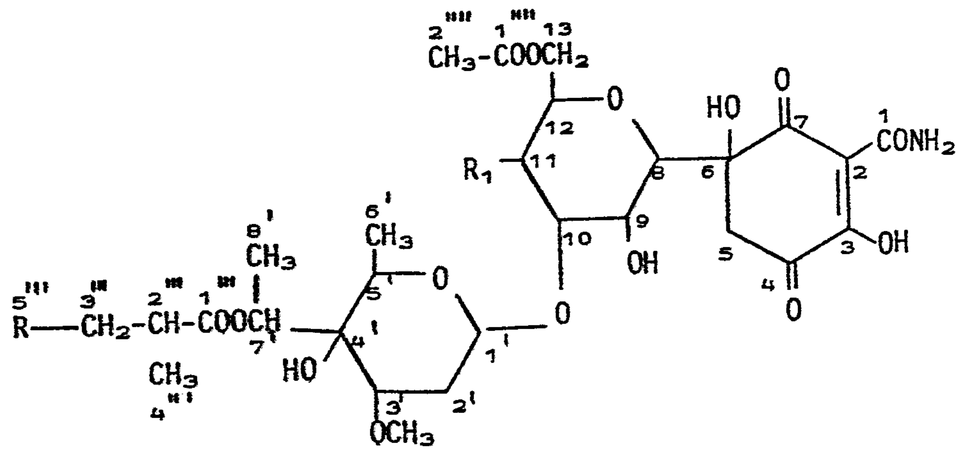

Paulomycin A R = $CH_3$

Paulomycin B R = H

$R_1$ =

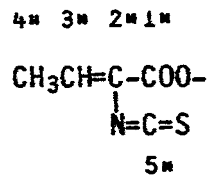

Antibiotic $273a_1\alpha$ R = $CH_3$

Antibiotic $273a_1\beta$ R = H

$R_1$ =

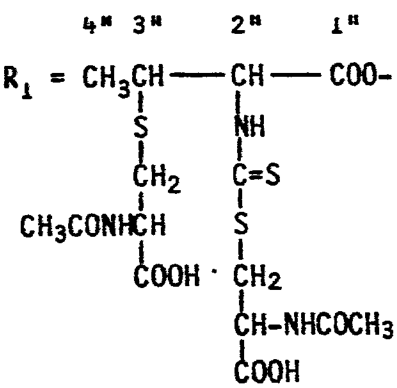

Antibiotic $273a_2\alpha$ R = $CH_3$

Antibiotic $273a_2\beta$ R = H

$R_1$ =

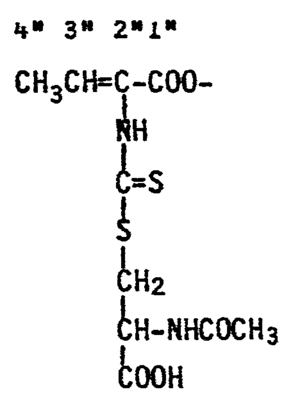

## CHART II

## Alkyl Esters of Antibiotic $273a_2$

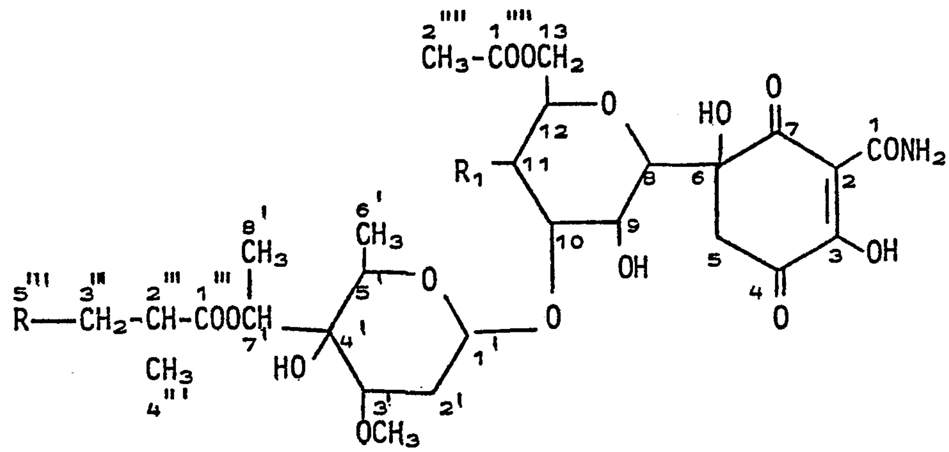

$R_1$ =

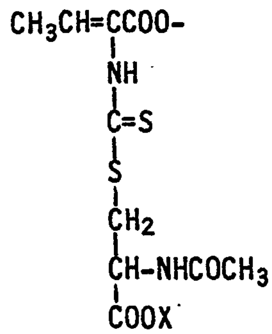

| | | | | |
|---|---|---|---|---|
| Antibiotic $273a_2\alpha$ | methyl ester | (1a) | $R=CH_3$ | $X=CH_3$ |
| Antibiotic $273a_2\beta$ | methyl ester | (1b) | R=H | $X=CH_3$ |
| Antibiotic $273a_2\alpha$ | ethyl ester | (2a) | $R=CH_3$ | X=ethyl |
| Antibiotic $273a_2\beta$ | ethyl ester | (2b) | R=H | X=ethyl |
| Antibiotic $273a_2\alpha$ | butyl ester | (3a) | $R=CH_3$ | X=butyl |
| Antibiotic $273a_2\beta$ | butyl ester | (3b) | R=H | X=butyl |
| Antibiotic $273a_2\alpha$ | octyl ester | (4a) | $R=CH_3$ | X=octyl |
| Antibiotic $273a_2\beta$ | octyl ester | (4b) | R=H | X=octyl |

0162568
4240

CHART III

$R_2SH$

5 $R_2 = -CH_2COOH$ (mercaptoacetic acid)

6 $R_2 = -CH_3CHCOOH$ (mercaptopropionic acid)

7 $R_2 =$ COOH (thiomalic acid)
CH-
$CH_2$
COOH

8 $R_2 = -CH_2CHCOOH$ (cysteine)
$NH_2$

9 $R_2 = -CH_2CH_2CHCOOH$ (homocysteine)
$NH_2$

10 $R_2 = H_2N·CH·CH_2CH_2COHNCHCOHNCH_2COOH$ (glutathione)
COOH $CH_2-$

11 $R_2 =$

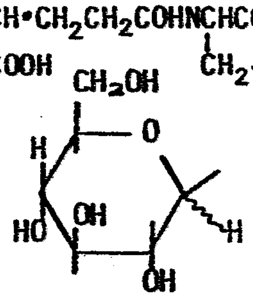

12 $R_2 = CH_2-$
CHOH
$CH_2OH$

13 $R_2 = CH_2-$
CHOH
CHOH
CHOH
$CH_2OH$

14 $R_2 = CH_2-$
CHOH
CHOH
CHOH
CHOH
$CH_2OH$

CHART IV

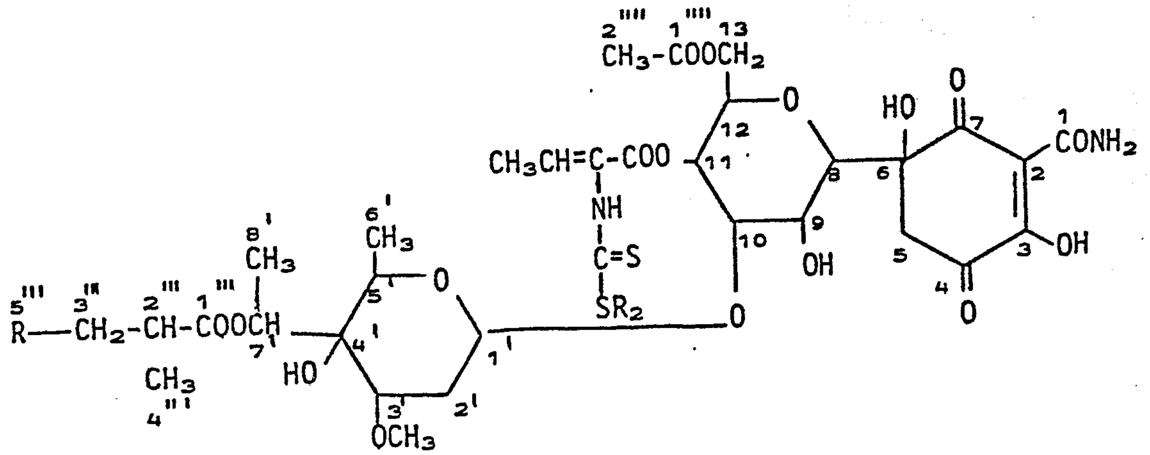

5a R = $CH_3$ $R_2$ = same as in 5
5b R = H $R_2$ = same as in 5
6a R = $CH_3$ $R_2$ = same as in 6
6b R = H $R_2$ = same as in 6
7a R = $CH_3$ $R_2$ = same as in 7
7b R = H $R_2$ = same as in 7
8a R = $CH_3$ $R_2$ = same as in 8
8b R = H $R_2$ = same as in 8
9a R = $CH_3$ $R_2$ = same as in 9
9b R = H $R_2$ = same as in 9
10a R = $CH_3$ $R_2$ = same as in 10
10b R = H $R_2$ = same as in 10
11a R = $CH_3$ $R_2$ = same as in 11
11b R = H $R_2$ = same as in 11
12a R = $CH_3$ $R_2$ = same as in 12
12b R = H $R_2$ = same as in 12
13a R = $CH_3$ $R_2$ = same as in 13
13b R = H $R_2$ = same as in 13
14a R = $CH_3$ $R_2$ = same as in 14
14b R = H $R_2$ = same as in 14

CLAIMS

1. A compound of the formula

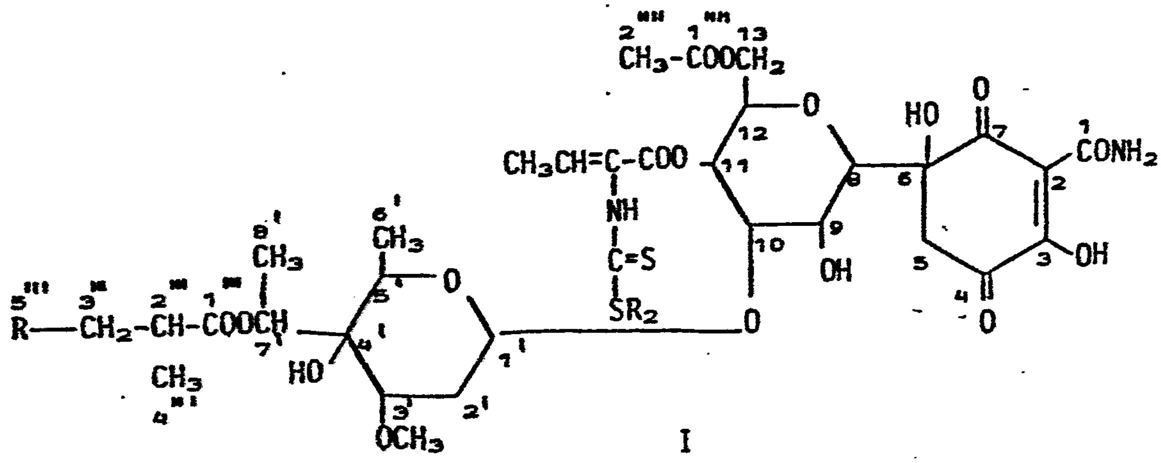

I

wherein R is hydrogen or methyl; and

$R_2$ is

(a) $-CH_2-CH(COOX_1)-NH-COCH_3$,

(b) $-CH_2COOX_2$,

(c) $-CH(CH_3)-COOX_2$,

(d) $-CH(COOX_2)-CH_2-COOX_2$,

(e) $-CH_2-CHNH_2-COOX_2$,

(f) $-CH_2-CH_2-CHNH_2-COOX_2$,

(g) $-CH_2-CH(CONH-CH_2-COOX_2)-NH-CO-CH_2-CH_2-CHNH_2-COOX_2$,

(h)

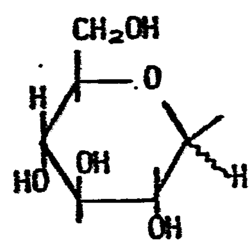

(i) $-CH_2-CHOH-CH_2OH$ or

(j) $-CH_2-(CHOH)_n-CH_2OH$;

wherein n is 3 or 4; $X_1$ is H, $C_{1-12}$ alkyl or a pharmacologically acceptable cation; and $X_2$ is H or a pharmacologically acceptable cation.

2. Antibiotic $273a_2$, the compounds of claim 1 wherein $R_2$ is $-CH_2-CH(COOH)-NH-COCH_3$.

3. Antibiotic $273a_{2\alpha}$, the compound of claim 2 wherein R is methyl.

4. Antibiotic $273a_{2\beta}$, the compound of claim 2 wherein R is hydrogen.

5. A compound of claim 1, wherein $R_2$ is $-CH_2-CH(COOX_1)-NH-COCH_3$ and $X_1$ is methyl, ethyl, butyl or octyl.

6. A process for preparing a compound of claim 1, which comprises reacting paulomycin A or paulomycin B with a compound of the formula $R_2SH$, wherein $R_2$ is as defined in claim 1.

7. A process according to claim 6, for preparing the compound as defined in claim 2, which comprises reacting approximately equimolar amounts of paulomycin A or paulomycin B and N-acetyl-L-cysteine.

8. A process for preparing antibiotic $273a_2$ as defined in claim 2, which comprises:

(a) extracting the clear filtrates of Streptomyces paulus Strain 273 (NRRL 12251) with ethyl acetate at a pH of approximately 3.0;

(b) adjusting the pH of the extract to approximately 5.5;

(c) recovering antibiotic $273a_2$ from the resultant aqueous phase; and, if desired,

(d) separating antibiotics $273a_1$ and $273a_2$ by counter-current distribution or HPLC.